# EUROPÄISCHE PATENTSCHRIFT

(11) **EP 2 572 094 B1**
(45) Veröffentlichungstag und Bekanntmachung des Hinweises auf die Patenterteilung: **08.08.2018**
(21) Anmeldenummer: 11709417.7
(22) Anmeldetag: 22.03.2011
(51) Int. Cl.: F02M 25/022, G01N 33/28, G01N 22/00, F02D 19/08

(54) **VORRICHTUNG ZUR BESTIMMUNG EINER ZUSAMMENSETZUNG EINES KRAFTSTOFFGEMISCHS MITTELS EINES VOM KRAFSTOFFGEMISCH DURCHSTRÖMTEN KOAXIALEN WELLENLEITERS**
DEVICE FOR DETERMINING THE COMPOSITION OF A FUEL MIXTURE BY MEANS OF A COAXIAL WAVEGUIDE THROUGH WHICH THE MIXTURE IS PASSED
DISPOSITIF POUR LA DÉTERMINATION D'UNE COMPOSITION D'UN MÉLANGE DE CARBURANT AU MOYEN D'UN GUIDE D'ONDES COAXIAL TRAVERSÉ PAR LE MÉLANGE DE CARBURANT

(30) Priorität: 17.05.2010 DE 102010029007
(43) Veröffentlichungstag der Anmeldung: 27.03.2013
(73) Patentinhaber: Robert Bosch GmbH, 70442 Stuttgart (DE)
(72) Erfinder: SOERGEL, Werner, 75181 Pforzheim (DE); SCHMIDT, Dirk, 73230 Kirchheim (DE)
(86) Internationale Anmeldenummer: PCT/EP2011/054318
(87) Internationale Veröffentlichungsnummer: WO 2011/144377

(56) Entgegenhaltungen:
- WO-A1-2005/109012
- BR-A- 0 204 519
- DE-A1-102006 034 884
- GB-A- 1 314 368
- SU-A1- 759 927
- US-A- 3 812 422
- US-A- 4 345 202
- US-A- 4 503 384
- US-A- 4 580 441
- US-A- 4 862 060
- US-A- 5 361 035
- US-A- 6 121 780
- US-A1- 2004 135 585
- US-A1- 2005 253 599
- SANTOS E J P: "Determination of ethanol content in gasoline: theory and experiment", MICROWAVE AND OPTOELECTRONICS CONFERENCE, 2003. IMOC 2003. PROCEEDINGS OF THE 2003 SBMO/IEEE MTT-S INTERNATIONAL 20-23 SEPT. 2003, PISCATAWAY, NJ, USA,IEEE, US, Bd. 1, 20. September 2003 (2003-09-20), Seiten 349-353, XP010669611, DOI: DOI:10.1109/IMOC.2003.1244884 ISBN: 978-0-7803-7824-7
- WEISS M ET AL: "A NOVEL METHOD OF DETERMINING THE PERMITTIVITY OF LIQUIDS", IEEE TRANSACTIONS ON INSTRUMENTATION AND MEASUREMENT, IEEE SERVICE CENTER, PISCATAWAY, NJ, US, Bd. 49, Nr. 3, 1. Juni 2000 (2000-06-01), Seiten 488-492, XP000937372, ISSN: 0018-9456, DOI: 10.1109/19.850381
- KENT M ET AL: "DESIGN NOTE; A simple flowthrough cell for microwave dielectric measurements", JOURNAL OF PHYSICS E. SCIENTIFIC INSTRUMENTS, IOP PUBLISHING, BRISTOL, GB, Bd. 22, Nr. 4, 1. April 1989 (1989-04-01), Seiten 269-271, XP020018983, ISSN: 0022-3735, DOI: 10.1088/0022-3735/22/4/012
- HENRIK H NISSEN ET AL: "TIME DOMAIN REFLECTOMETRY DEVELOPMENTS IN SOIL SCIENCE: II. COAXIAL FLOW CELL FOR MEASURING EFFLUENT ELECTRICAL CONDUCTIVITY", SOIL SCIENCE, Bd. 168, Nr. 2, 1. Februar 2003 (2003-02-01), Seiten 84-89, XP55006073, DOI: 10.1097/01.ss.0000055303.23789.39
- KURT C LAWRENCE ET AL: "Flow-Through Coaxial Sample Holder Design for Dielectric Properties Measurements from 1 to 350 MHz", IEEE TRANSACTIONS ON INSTRUMENTATION AND MEASUREMENT, IEEE SERVICE CENTER, PISCATAWAY, NJ, US, Bd. 47, Nr. 2, 1. April 1998 (1998-04-01), XP011024484, ISSN: 0018-9456
- HUDIARA I S ET AL: "Microwave properties of commercial petrol over 900 MHz to 9 GHz", JOURNAL OF THE INSTITUTION OF ELECTRONICS ANDTELECOMMUNICATION ENGINEERS, ENGINEERS, NEW DEHLI, Bd. 40, Nr. 5-6, 1. September 1994 (1994-09-01), Seiten 291-292, XP009151679, ISSN: 0377-2063

## Beschreibung

### Stand der Technik

Aus dem Stand der Technik sind Verfahren und Vorrichtungen zur Bestimmung von Zusammensetzungen von Kraftstoffgemischen bekannt. Beispielsweise werden in Kraftfahrzeugen zunehmend Kraftstoffgemische eingesetzt, welche neben den eigentlichen Mineralöl-Kraftstoffen eine Beimischung an Ethanol und/oder anderen Alkoholen verarbeiten können. Üblicherweise werden dabei die Parameter der Motorsteuerung des Kraftfahrzeugs auf die Zusammensetzung des Kraftstoffgemischs angepasst. Zu diesem Zweck werden jedoch in vielen Fällen Kenntnisse über die Zusammensetzung des Kraftstoffgemischs, insbesondere eines Ethanol-Kraftstoff-Mischungsverhältnisses, vorausgesetzt. Ein bekanntes Messverfahren ist dabei die Messung einer Absorption, Transmission oder Reflexion von Mikrowellen durch das Kraftstoffgemisch. Ein Beispiel einer Vorrichtung, welche zu diesem Zweck eingerichtet ist, ist aus DE 34 12 704 A1 bekannt. Weiterhin ist aus der nachveröffentlichten DE 10 2008 044 383.2 ein Verfahren zur Bestimmung einer Zusammensetzung eines Kraftstoffgemischs bekannt, bei welchem über einen größeren Frequenzbereich hinweg ein charakteristischer Verlauf einer bestimmten Antwort auf eine Mikrowelleneinstrahlung zur Ermittlung der Eigenschaften des Kraftstoffgemischs erfasst und daraus auf die Zusammensetzung des Kraftstoffgemischs geschlossen wird. Ein Vorteil des in DE 10 2008 044 383.2 beschriebenen Verfahrens besteht insbesondere darin, dass die Genauigkeit durch Verwendung größerer Frequenzbereiche gesteigert werden kann, so dass beispielsweise Alkohol-KraftstoffGemische, welche zusätzlich noch einen Wasseranteil und/oder Additive enthalten, erheblich genauer charakterisiert werden können als mit herkömmlichen Verfahren. Es besteht jedoch ein Verbesserungspotenzial hinsichtlich der Einkopplung und Auskopplung der Mikrowellensignale in den bzw. aus dem Kraftstoff. DE102006034884 offenbart eine Vorrichtung zur Bestimmung der Zusammensetzung eines Fluids, die einen Koaxialleitungsresonator bestehend aus einem löchrigen, becherförmigen Aussenleiter und einem mit dem Aussenleiter am Ende kurzgeschlossenen Innenleiter aufweist. Die vom in das zu untersuchende Fluid eingetauchten Koaxialleiter reflektierten Mikrowellen werden ausgewertet, um die Zusammensetzung zu bestimmen.

### Offenbarung der Erfindung

Es wird eine Vorrichtung vorgeschlagen, welche zur Bestimmung einer Zusammensetzung eines Kraftstoffgemischs einsetzbar sind. Auf diese Weise können insbesondere dielektrische Eigenschaften von Kraftstoffen zur Bestimmung des Ethanolgehalts von Kraftstoffgemischen herangezogen werden. Alternativ oder zusätzlich können andere Anteile an dem Kraftstoffgemisch quantitativ bestimmt werden, beispielsweise Wasseranteile und/oder Anteile von Additiven.

Die erfindungsgemässe Vorrichtung ist in Anspruch 1 definiert.

Das Gehäuseelement ist vorzugsweise aus einem kraftstoffbeständigen Material hergestellt, beispielsweise aus einem kraftstoffbeständigen Metall, wie beispielsweise einem Edelstahl oder einem anderen metallischen Material. Ein bevorzugtes Material ist insbesondere Edelstahl vom Typ 1.4301.

Unter Mikrowellensignalen sind dabei allgemein elektromagnetische Hochfrequenzsignale zu verstehen, welche vorzugsweise in einem Frequenzbereich von oberhalb von 100 MHz oder sogar 300 MHz liegen, beispielsweise in einem Frequenzbereich zwischen 300 MHz und 300 GHz, insbesondere zwischen 300 MHz und 20 GHz, vorzugsweise zwischen 500 MHz und 10 GHz und besonders bevorzugt im Bereich von 0,5 und 8,5 GHz.

Die Achse des Bechers erstreckt sich quer zu einer Strömungsrichtung des Kraftstoffgemischs durch die Vorrichtung, d.h. quer zur Strömungsrohrachse. Beispielsweise kann sich die Becherachse im Wesentlichen senkrecht, d.h. beispielsweise unter einem Winkel von 90°±20°, vorzugsweise 90°±10° und besonders bevorzugt 90°±5°, zu einer Strömungsrohrachse erstrecken. Der Becher kann insbesondere (vorzugsweise außerhalb des Strömungsquerschnitts) einen geschlossenen Becherboden aufweisen. Der Becher kann somit als an einem Ende durch den Becherboden geschlossener Hohlzylinder beschrieben werden. Der Becher ist elektrisch leitend mit dem Innenleiter verbunden sein, insbesondere im Bereich des Becherbodens. Beispielsweise kann der Innenleiter in den Becherboden hineinragen, hineingesteckt sein (beispielsweise wenn der Innenleiter als Steckerstift ausgestaltet ist) oder auf andere Weise elektrisch leitend mit dem Becherboden verbunden sein.

Das Gehäuseelement, insbesondere der Becher, kann insbesondere eine Mehrzahl von Bohrungen aufweisen, durch welche ein Einströmen von Kraftstoff ins Innere des Bechers ermöglicht wird sowie ein Ausströmen von Kraftstoff aus dem Inneren des Bechers. Vorzugsweise weist der Becher also eine Mehrzahl von Bohrungen auf. Die Bohrungen weisen vorzugsweise einen Durchmesser auf, welcher erheblich kleiner ausgestaltet ist als die kleinste Wellenlänge der eingekoppelten Mikrowellen in dem Kraftstoffgemisch. Auf diese Weise kann eine Auskopplung von Mikrowellen, welche sich zwischen dem Innenleiter und dem Becher bzw. der Innenwand des Bechers ausbilden, vermieden werden. Vorzugsweise weisen die Bohrungen einen Durchmesser auf, welcher kleiner ist als 2 mm, insbesondere kleiner als 1 mm. Beispielsweise ist für E100, also Kraftstoff mit einem nominellen Ethanolanteil von 100%, bei einer Frequenz f=19 GHz die minimale Wellenlänge λ=7 mm. Die Bohrungen können dabei grundsätzlich einen beliebigen Querschnitt aufweisen. Besonders bevorzugt sind runde Querschnitte. Auch nicht-runde Querschnitte sind jedoch grundsätzlich denkbar. Unter einem "Durchmesser" bei nicht-runden Querschnitten, beispielsweise polygonalen Querschnitten, ist unter einem "Durchmesser" ein "Äquivalentdurchmesser" zu verstehen, beispielsweise ein Durchmesser eines Kreises mit gleicher Öffnungsfläche.

Die Verbindungsvorrichtung soll zum Ein- und/oder Auskoppeln von Mikrowellensignalen dienen. Insbesondere kann diese Verbindungsvorrichtung einen Stecker und/oder eine Steckverbindung aufweisen. Besonders bevorzugt sind hierbei genormte Steckverbindungen. Beispielsweise können Koaxialstecker verwendet werden, bei welchen mindestens ein Kontakt zur Beaufschlagung des Innenleiters mit Mikrowellensignalen vorgesehen ist, also zur Einkopplung und/oder Auskopplung von Mikrowellensignalen. Der Kontakt wird bei einem Koaxialstecker von mindestens einem abschirmenden Steckerteil umgeben. Beispielsweise können hierbei SMA-RP-Steckverbinder eingesetzt werden.

Vorzugsweise sind die Bohrungen des Bechers jeweils der Strömungsrichtung zuweisend ausgerichtet, so dass der Becher der Kartusche ohne Umleitung der Strömung von dem Kraftstoffgemisch durchströmbar ist. Die Aufnahme umfasst eine Erweiterung in dem Gehäuse, mit mindestens einer Aufnahmebohrung, in welche die Kartusche quer zur Strömungsrichtung einsteckbar ist. Die einsteckbare Aufnahme kann dabei reversibel oder auch permanent erfolgen. Nach Einstecken der Kartusche kann beispielsweise eine Fixierung der Kartusche in der Aufnahme erfolgen, beispielsweise durch eine Verschraubung, Verstemmung, Klemmringe oder andere Arten von Fixierungen. Weiterhin kann die Einsteckkartusche insbesondere mittels mindestens eines Dichtelements gegenüber dem Strömungsrohrabschnitt abgedichtet sein. Beispielsweise können zu diesem Zweck ein, zwei, vier oder mehrere O-Ringe vorgesehen sein. Die oben beschriebene Verbindungsvorrichtung kann beispielsweise auf einer Stirnseite der Kartusche außerhalb des Strömungsrohrabschnitts angeordnet sein, so dass beispielsweise der Strömungsrohrabschnitt, die Kartusche und die Verbindungsvorrichtung, beispielsweise die Steckverbindung, eine T-Konfiguration bilden. Weiterhin kann der Strömungsrohrabschnitt beispielsweise zwei oder mehr Anschlüsse aufweisen, beispielsweise einen Zulaufanschluss und einen Ablaufanschluss. Diese Anschlüsse können genormt ausgestaltet sein, beispielsweise in Form von kostengünstigen, hydraulischen genormten Anschlüssen, beispielsweise nach der SAE-Norm.

Der Strömungsrohrquerschnitt ist in dem Messbereich gegenüber dem Strömungsrohrquerschnitt außerhalb des Messbereichs aufgeweitet. Dies bedeutet, dass der Messbereich in einem größeren Querschnitt durchströmt wird als sonstige Bereiche der Vorrichtung, beispielsweise Bereiche des Strömungsrohrs außerhalb des Messbereichs. In anderen Worten kann die Strömung im Messbereich aufgeweitet sein. Die Aufweitung erfolgt dadurch, dass in dem Messbereich eine, zwei oder mehr Schalen vorgesehen sind, und zwar als Bestandteil der Kartusche, welche für die Aufweitung in dem Messbereich sorgen, und zwar für eine kontinuierliche Aufweitung, also eine Aufweitung mit einem im Wesentlichen linearen Verlauf. Die Aufweitungsschalen können beispielsweise Totvolumina am Übergang zwischen dem Strömungsrohrabschnitt und dem Messbereich, beispielsweise am Übergang zwischen dem Strömungsrohrabschnitt und der Einsteckkartusche, vermeiden. Die Schalen können beispielsweise ringförmig ausgestaltet sein und beispielsweise ebenfalls konzentrisch um den Innenleiter in der Einsteckkartusche aufgenommen sein. Die Schalen können beispielsweise aus einem Kunststoffmaterial, beispielsweise einem kraftstoffbeständigen Kunststoffmaterial, beispielsweise einem fluorierten Polyethylen, hergestellt sein. Bei der Bestimmung einer Zusammensetzung eines Kraftstoffgemischs kommt ein Verfahren zum Einsatz, bei welchem über einen Frequenzbereich hinweg Mikrowellensignale unter Verwendung einer Vorrichtung gemäß einer oder mehreren der oben beschriebenen Ausgestaltungen in das Kraftstoffgemisch eingekoppelt werden. Dabei werden Antwortsignale unter Verwendung der Vorrichtung empfangen. Antwortsignale können dabei beispielsweise reflektierte und/oder transmittierte Signale sein, welche beispielsweise ebenfalls über die Verbindungsvorrichtung ausgekoppelt werden können. Aus einem Vergleich der eingekoppelten Mikrowellensignale und der Antwortsignale wird dabei mindestens eine Kenngröße in Abhängigkeit von der Mikrowellenfrequenz der eingekoppelten Mikrowellensignale bestimmt. Aus einem Verlauf der Kenngröße über die Mikrowellenfrequenz wird dabei auf die Zusammensetzung des Kraftstoffgemischs geschlossen.

Dabei lässt sich grundsätzlich das in DE 10 2008 044 383.2 beschriebene Verfahren einsetzen. Auch andere Verfahren sind jedoch grundsätzlich möglich. Das Antwortsignal stellt dabei allgemein eine Reaktion des Kraftstoffgemischs auf die Einstrahlung des eingekoppelten Signals dar. Beispielsweise kann es sich dabei allgemein um transmittierte, reflektierte, remittierte oder emittierte Signale oder um Kombinationen mehrerer Arten dieser Signale handeln. Alternativ oder zusätzlich können auch aus der Absorption, d.h. einem Fehlen von Signalanteilen, Rückschlüsse gezogen werden. Die Kenngröße kann dabei grundsätzlich eine beliebige Größe umfassen, welche aus den eingekoppelten Mikrowellensignalen und den Antwortsignalen gebildet wird. Dabei können beispielsweise auch Linearkombinationen aus Amplituden und/oder Phasen der eingekoppelten Signale und entsprechenden Größen der Antwortsignale gebildet werden. Beispielsweise kann eine Differenz zwischen einer Amplitude der eingekoppelten Mikrowellenstrahlung und einer Amplitude der Antwortmikrowellenstrahlung und eine Differenz aus der Phase der eingekoppelten Mikrowellenstrahlung und der Phase der ausgekoppelten Mikrowellenstrahlung gebildet werden. Die Differenzen können dann jeweils die Kenngröße bilden und/oder einen Teil dieser Kenngröße. Entsprechend der Art des Vergleichs der eingekoppelten Signale und der Antwortsignale kann die Kenngröße beispielsweise mindestens eine der folgenden Kenngrößen umfassen: eine Permitivität, insbesondere komplexe Permitivität; eine Permitivitätszahl, insbesondere eine komplexe Permitivitätszahl; eine Absorption, insbesondere eine komplexe Absorption; eine Transmission, insbesondere eine komplexe Transmission. Unter komplexen Größen ist dabei jeweils eine Größe zu verstehen, welche eine Amplitude und eine Phase beinhaltet. Die Permitivität, welche häufig auch mit dem Buchstaben ε bezeichnet wird, beschreibt die Durchlässigkeit von Materialien für elektrische Felder. Sie ist eine Materialeigenschaft von Dielektrika oder zumindest nur schwach elektrisch leitfähigen Materialien, welche sich bei der Beaufschlagung dieser Materialien mit elektrischen Feldern äußert. Sie stellt die Proportionalitätskonstante zwischen der elektrischen Flussdichte D und dem elektrischen Feld dar. Die Permitivitätszahl, häufig auch als εᵣ bezeichnet oder auch als relative Permitivität, ist das Verhältnis der Permitivität ε zur elektrischen Feldkonstante ε₀ (Permitivität des Vakuums).

Bei dem Verfahren werden Mikrowellensignale über einen Frequenzbereich hinweg eingekoppelt. Dies bedeutet, dass Mikrowellensignale mit mindestens zwei Mikrowellenfrequenzen eingekoppelt werden. Diese zwei Mikrowellenfrequenzen können nacheinander und/oder gleichzeitig eingekoppelt werden. Die Mikrowellenfrequenzen decken vorzugsweise einen Frequenzbereich von mindestens 100 MHz ab. Dabei kann dieser Frequenzbereich durch die mindestens zwei Mikrowellenfrequenzen kontinuierlich oder auch in regelmäßigen oder unregelmäßigen Schritten abgedeckt sein. Besonders bevorzugt ist es, wenn die eingekoppelten Mikrowellensignale eine Ultrabreitband-Mikrowellenstrahlung umfassen. Unter einer Ultrabreitband-Mikrowellenstrahlung (englisch Ultra Wide Band, UWB) wird dabei eine Mikrowellenstrahlung im Sinne der obigen Definition verstanden, welche einen extrem großen Frequenzbereich nutzt, mit einer Bandbreite von mindestens 500 MHz. Wie oben dargestellt, wird bei dem vorgeschlagenen Verfahren aus dem Verlauf der Kenngröße über die Mikrowellenfrequenz auf die Zusammensetzung des Kraftstoffgemischs geschlossen. Dieser Verlauf kann beispielsweise in einem einzelnen Schritt bestimmt werden, beispielsweise indem die Kenngrößen über den Frequenzbereich hinweg gleichzeitig und/oder nacheinander bestimmt werden, oder es kann eine iterative oder schrittweise Bestimmung der Kenngrößen über den Frequenzbereich hinweg erfolgen.

Zur Auswertung des gemessenen Verlaufs der Kenngröße und somit zur Bestimmung der Zusammensetzung des Kraftstoffgemischs können grundsätzlich beliebige analytische, semiempirische oder empirische Verfahren herangezogen werden. Allgemein kann unter der Bestimmung der Zusammensetzung des Kraftstoffgemischs beispielsweise die Bestimmung einer Konzentration einer einzelnen oder mehrerer Komponenten dieses Kraftstoffgemischs und/oder ein Mischungsverhältnis verstanden werden. Dementsprechend kann die Bestimmung der Zusammensetzung des Kraftstoffgemischs vollständig, im Sinne einer vollständigen Analyse, oder lediglich teilweise erfolgen, beispielsweise indem lediglich die Konzentration und/oder das Mischungsverhältnis einer einzelnen Komponente und/oder mehrerer Komponenten bestimmt werden. Beispielsweise können Referenzverläufe der Kenngrößen analytisch, empirisch oder semiempirisch bestimmt werden. So kann das Kraftstoffgemisch beispielsweise mindestens zwei, vorzugsweise drei, vier oder mehr Komponenten umfassen, wobei jeweils Referenzverläufe der Kenngröße über die Frequenz der eingekoppelten Mikrowellensignale für diese Komponenten zumindest teilweise bekannt sind. Beispielsweise können diese Referenzverläufe dadurch ermittelt werden, dass die jeweiligen Komponenten als Reinstoffe oder als im Wesentlichen reine Stoffe mittels des oben beschriebenen Verfahrens untersucht und dabei Kennlinienverläufe aufgenommen werden. Diese Kennlinienverläufe der einzelnen Komponenten können dann als Referenzverläufe für die jeweilige Komponente beispielsweise in einem Speicher hinterlegt sein. Bei der eigentlichen Messung der Zusammensetzung des Kraftstoffgemischs kann dann aus dem gemessenen Verlauf der tatsächlichen Kenngröße mittels der bekannten Referenzverläufe auf die Zusammensetzung des Kraftstoffgemischs geschlossen werden. Dieser Rückschluss auf die Zusammensetzung kann beispielsweise durch einen Einzelvergleich des gemessenen Verlaufs mit den Referenzverläufen und/oder einer Interpolation oder Extrapolation derselben erfolgen. Auch andere Verfahren sind denkbar. So kann beispielsweise eine Linearkombination der Referenzverläufe gebildet werden, um auf diese Weise die Linearkombination der Referenzverläufe an den gemessenen Verlauf anzupassen. Aus den bei dieser Anpassung an den gemessenen Verlauf bestimmten Koeffizienten der Linearkombination, welche beispielsweise nach bekannten Anpassungsverfahren bestimmt werden können, kann dann auf die Anteile der einzelnen Komponenten geschlossen werden. Auch andere Möglichkeiten sind jedoch denkbar, beispielsweise indem eine Vielzahl von Kombinationen und entsprechende Referenzverläufe für verschiedene Kraftstoffgemischzusammensetzungen in einem Speicher hinterlegt werden und dementsprechend aus dem gemessenen Verlauf der Kenngröße Referenzverläufe mit der größtmöglichen Übereinstimmung herausgesucht werden. Auch auf diese Weise lässt sich auf die Zusammensetzung des Kraftstoffgemischs schließen. Zusätzlich kann die Temperatur nahe dem Kraftstoffvolumen gemessen werden um die TemperaturAbhängigkeit der Messgrößen zu kompensieren.

Die oben beschriebene Vorrichtung weist gegenüber bekannten Vorrichtungen zahlreiche Vorteile auf. So lässt sich ein von dem Kraftstoffgemisch durchströmter Messfühler realisieren, der aus mehreren Einzelteilen zusammengesetzt sein kann, wobei eine einfache und kostengünstige Herstellung möglich ist, insbesondere unter Verwendung der oben beschriebenen Einsteckkartusche. Weiterhin lässt sich ein Messfühler mit einem reduzierten Volumen ohne Totvolumen realisieren. Der Messfühler kann elektromagnetisch neutral ausgestaltet werden, ohne die Umgebung des Messfühlers mit Mikrowellenstrahlung zu beaufschlagen. Der Messfühler lässt sich kostengünstig ausgestalten und beispielsweise mit SAE-Norm-konformen hydraulischen Anschlüssen versehen.

### Kurze Beschreibung der Figuren

Ausführungsbeispiele der Erfindung sind in den Figuren dargestellt und werden in der nachfolgenden Beschreibung näher erläutert.

Es zeigen:
- Figur 1: einen Querschnitt durch eine erfindungsgemäße Vorrichtung zur Bestimmung einer Zusammensetzung eines Kraftstoffgemischs mit einer Schnittebene parallel zu einer Strömungsrichtung;
- Figur 2: eine perspektivische Darstellung der Vorrichtung gemäß Figur 1; und
- Figur 3: einen Querschnitt durch einen Messbereich der Vorrichtung gemäß Figur 1 mit einer Schnittebene senkrecht zur Schnittebene gemäß Figur 1.

### Ausführungsbeispiele

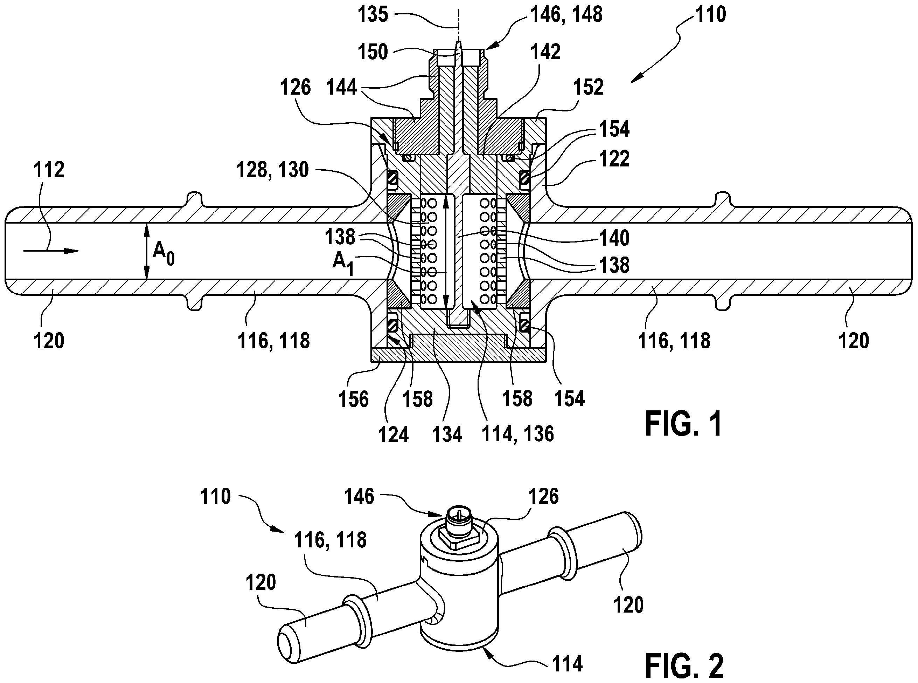

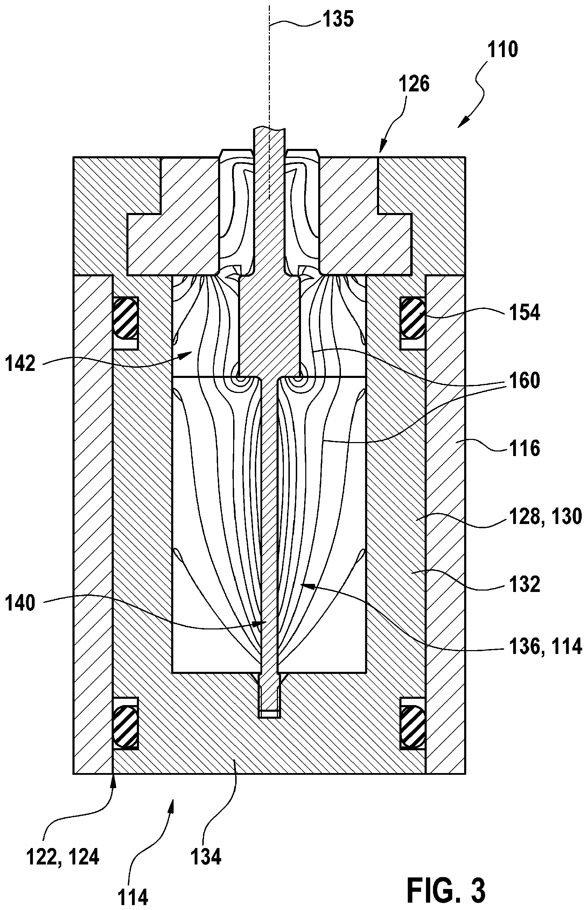

In den Figuren 1 bis 3 ist ein Ausführungsbeispiel einer erfindungsgemäßen Vorrichtung 110 zu Bestimmung einer Zusammensetzung eines Kraftstoffgemischs dargestellt. Diese kann insbesondere zur Bestimmung eines Ethanolanteils in einem Kraftstoff eingesetzt werden. Die Vorrichtung 110 ist in Figur 1 in einer Schnittdarstellung mit einer Schnittebene parallel zu einer Strömungsrichtung 112 des Kraftstoffgemischs dargestellt, in Figur 2 in einer perspektivischen Darstellung, und in Figur 3 ist ein schematischer Querschnitt durch die Vorrichtung 110 durch einen Messbereich 114 mit einer Schnittebene senkrecht zur Schnittebene in Figur 1 gezeigt, wobei die schematische Darstellung gemäß Figur 3 symbolisch einer Verdeutlichung eines Feldlinienverlaufs dienen soll.

Die Vorrichtung 110 umfasst, wie in Figur 1 erkennbar ist, ein Gehäuse 116. Das Gehäuse 116 umfasst einen Strömungsrohrabschnitt 118 mit hydraulischen Anschlüssen 120, beispielsweise zum Anschließen einer Kraftstoffleitung. Die hydraulischen Anschlüsse 120 können beispielsweise genormt ausgestaltet sein. Weiterhin umfasst das Gehäuse 116 in dem Messbereich 114, in diesem Ausführungsbeispiel exemplarisch symmetrisch von den hydraulischen Anschlüssen 120 umgeben, eine Aufnahme 122, beispielsweise mit einer zylindrischen Aufnahmebohrung 124. In dieser Aufnahmebohrung 124 ist, im Wesentlichen senkrecht zur Strömungsrichtung 112, eine Einsteckkartusche 126 eingebracht, welche in diesem Ausführungsbeispiel die eigentliche Messvorrichtung darstellt. Diese Einsteckkartusche 126 umfasst ein durchströmbares Gehäuseelement 128 in Form eines metallischen Bechers 130. Dieser Becher umfasst eine zylindrische Becherwand 132 und einen Becherboden 134. Eine Achse 135 des Bechers 130 bzw. der Einsteckkartusche 126, welche sich senkrecht zum Becherboden 134 und parallel zu der Becherwand 132 erstreckt, steht im Wesentlichen senkrecht auf die Strömungsrichtung 112. Die Becherwand 132 umschließt ein Messvolumen 136 des Messbereichs 114. Die Becherwand 132 weist, zumindest entgegen und abgewandt der Strömungsrichtung 112, eine Mehrzahl von Bohrungen 138 auf, welche in diesem Ausführungsbeispiel als Bohrungsmatrix ausgestaltet sind. Durch diese Bohrungen 138, welche als Strömungsbohrungen fungieren, kann Kraftstoffgemisch in Figur 1 auf der linken Seite in das Messvolumen 136 des Messbereichs 114 eintreten und auf der rechten Seite in Figur 1 aus diesem wieder austreten oder umgekehrt. Die Einsteckkartusche 126 kann zum Beispiel an der Oberseite mit einer zusätzlichen Sacklochbohrung versehen werden, die als Aufnahme eines Temperatursensors dienen kann.

Das Gehäuseelement 128 bzw. der Becher 130 umschließen in dem dargestellten Ausführungsbeispiel konzentrisch einen stiftförmigen Innenleiter 140. Dieser wird also in dem Messvolumen 136 von Kraftstoffgemisch umspült. Der Innenleiter 140 ist an seinem unteren Ende mit dem metallischen Becherboden 134 verbunden, indem der Innenleiter 140 in diesen Becherboden 134 eingesteckt ist. Oberhalb des Messvolumens 136 ist der Innenleiter 140 in einem Dielektrikum 142 eingebettet, beispielsweise einem Kunststoff, durch welchen der Innenleiter 140 elektrisch gegenüber einem Steckergehäuse 144 getrennt ist. Das Steckergehäuse 144 ist Teil einer Verbindungsvorrichtung 146, welche in diesem Fall in Form eines Koaxialsteckers 148 ausgestaltet ist. Diese umfasst neben dem Steckergehäuse 144 einen Kontakt 150 des Innenleiters 140. Beispielsweise kann der Koaxialstecker 150 als SMA-Stecker ausgestaltet sein.

Das Steckergehäuse 144 ist an seinem oberen Ende mit einem Becherrand 152 des Bechers 130 verschraubt. Das Steckergehäuse 144 und der Becher 130 sind also vorzugsweise elektrisch miteinander verbunden. Das Steckergehäuse 144 und der Becher 130 sind dadurch elektrisch miteinander verbunden. Der Becher 130 selbst kann beispielsweise mit einem Außenleiter oder Steckergehäuse der Verbindungsvorrichtung 146, beispielsweise eines Koaxialsteckers, verbunden sein. Auf diese Weise bildet der Innenleiter mit dem Becher vorzugsweise eine kurzgeschlossene Koaxialleitung. Zwischen dem eingeschraubten Steckergehäuse 144 und dem Becher 130 kann zusätzlich, wie in Figur 1 dargestellt, mindestens ein Dichtelement 154 eingebracht sein, beispielsweise in Form eines oder mehrerer O-Ringe, beispielsweise aus einem kraftstoffbeständigen Material. Weiterhin können auch zwischen der Einsteckkartusche 126 und der Wand der Aufnahme 122 des Gehäuses 116 ein oder mehrere Dichtelemente 154 vorgesehen sein, beispielsweise in entsprechenden Ringnuten des Bechers 130, wie in Figur 1 angedeutet. An der der Verbindungsvorrichtung 146 gegenüberliegenden Stirnseite der Einsteckkartusche 126 kann diese beispielsweise durch eine Verschlusssicherung 156 gesichert sein, beispielsweise ebenfalls eine Verschraubung, so dass die Einsteckkartusche 126 fest in der Aufnahme 122 verankert ist.

Im Bereich der Einsteckkartusche 126 findet weiterhin eine Aufweitung des Strömungsquerschnitts von einem ursprünglich kleinen Strömungsquerschnitt A₀ im Bereich der hydraulischen Anschlüsse 120 hin zu einem Strömungsquerschnitt A₁ im Bereich des Messvolumens 136 statt. Diese Aufweitung erfolgt in dem dargestellten Ausführungsbeispiel kontinuierlich durch Schalen 158, welche Bestandteil der Einsteckkartusche 126 sein können und welche beispielsweise aus einem Kunststoffmaterial hergestellt sein können.

Die in den Figuren 1 und 2 dargestellte Vorrichtung 110 weist einen vergleichsweise einfach herzustellenden und dennoch effektiven Aufbau auf. Innerstes Bauteil der als Messfühler ausgestalteten Vorrichtung 110 ist der in diesem Fall zylindrisch ausgestaltete Becher 130, welcher aus korrosionsbeständigem und kraftstoffverträglichem Metall hergestellt sein kann. In diesem ist vorzugsweise nach dem koaxialen Prinzip der Innenleiter 140 aufgenommen, so dass die Einsteckkartusche 126 prinzipiell ein koaxialer Messfühler ist. Der Innenleiter 140 ist leitend mit dem Becherboden 134 verbunden, wobei der Becher selbst aufgrund der Matrix an Bohrungen 138 durchströmbar ist. Die Bohrungen 138 können insgesamt von ihrem Durchflussquerschnitt her derart bemessen sein, dass der Durchflussquerschnitt A₀ durch die Gesamtzahl der Bohrungen 138 auf einer Seite des Bechers 130 erhalten bleibt. Zur Aufweitung der Strömung wird der Querschnitt des kraftstoffbeständigen und vorzugsweise SAE-Norm-konformen Anschlusses A₀ kontinuierlich auf den Bohrungsmatrixquerschnitt mittels der eingelegten Schalen 158 aufgeweitet. Die Bohrungsdurchmesser der einzelnen Bohrungen 138 sind dabei vorzugsweise klein gegenüber der minimalen, im Kraftstoffgemisch auftretenden Wellenlänge. Damit wird eine unerwünschte Abstrahlung in die Umgebung vermieden. Das Messprinzip der Vorrichtung 110 gemäß den Figuren 1 und 2 soll exemplarisch an der Schnittdarstellung gemäß Figur 3 erläutert werden. Diese Darstellung zeigt, wie oben ausgeführt, eine Schnittdarstellung senkrecht zur Schnittebene in Figur 1, durch die Achse 135. Es sei darauf hingewiesen, dass es sich hierbei lediglich um eine schematische Darstellung handelt, aufgrund welcher der Verlauf der elektrischen Feldlinien 160 bei einer einzelnen Frequenz erläutert werden kann.

Die Vorrichtung 110 kann über die Verbindungsvorrichtung 146, beispielsweise über einen koaxialen, genormten Steckverbinder (z.B. SMA-RP), mit einer Hochfrequenz (HF)-Sensorschaltung verbunden sein. Über diese kann die Vorrichtung 110 mit Mikrowellensignalen oder allgemein Hochfrequenzsignalen verschiedener Frequenzen beaufschlagt werden, und es können Antwortsignale über dieselbe Verbindungsvorrichtung 146 erfasst werden. Die Verbindungsvorrichtung 146 dient also vorzugsweise gleichzeitig zum Einkoppeln von Mikrowellensignalen und zum Auskoppeln von Antwortsignalen. Jeweils aus eingekoppeltem Signal und Antwortsignal können eine oder mehrere Kenngrößen ermittelt werden, sowie der Verlauf dieser Kenngrößen über die Frequenz der eingekoppelten Strahlung. Eine wesentliche Kenngröße ist, wie oben beschrieben, die Permitivität. Beispielsweise kann aus einer Differenz (Amplitude und Phase) zwischen ausgesendetem bzw. eingekoppeltem und empfangenem Signal bzw. Antwortsignal auf die Permitivität des sich im Messvolumen befindlichen Kraftstoffgemischs geschlossen werden. Der Verlauf der Permitivität und/oder einer oder mehrerer alternativer Kenngrößen über die Frequenz wird zur Identifizierung und Quantifizierung von Einzelkomponenten des Kraftstoffgemischs herangezogen. Wie oben dargestellt kann hierzu beispielsweise ein Vergleich mit Referenzkurven erfolgen, beispielsweise durch eine Anpassung von Linearkoeffizienten, beispielsweise unter Minimierung von Fehlerquadraten oder unter Verwendung ähnlicher Anpassungsverfahren.

Das mit Kraftstoffgemisch gefüllte Messvolumen 136 in Form des gefüllten Koaxialleitungsabschnitts hat, abhängig von den Verlusteigenschaften des Kraftstoffgemischs (beschrieben beispielsweise durch den Imaginärteil der Permitivität des Kraftstoffgemischs) eine dämpfende und abhängig von dem Realteil der Permitivität eine verzögernde Wirkung auf vorlaufende und rücklaufende Wellen. Die rücklaufende Welle erfährt am Ende der kurzgeschlossenen Koaxialleitung im Bereich des Becherbodens 134 einen definierten Phasensprung von beispielsweise 180°.

Die Kenngröße wird bei verschiedenen Frequenzen über einen möglichst großen Frequenzbereich hinweg ermittelt. Beispielsweise kann dies durch den Einsatz eines Ultra-Breitband-Sensors (UWB) erfolgen. Durch den Einsatz eines UWBs wird die Permitivität nicht nur bei einzelnen Frequenzlinien ermittelt, sondern deren Verlauf über den gesamten gemessenen Frequenzbereich, beispielsweise einen Frequenzbereich von 1,5 GHz bis 8,5 GHz. Hierdurch wird der Informationsgehalt der Messung erheblich erhöht. Insgesamt lässt sich auf diese Weise eine einfach herzustellende, kostengünstige und einfach zu handhabende sowie zuverlässig abdichtende Messvorrichtung schaffen, welche beispielsweise in Kraftfahrzeugen eingesetzt werden kann, insbesondere in Ottomotoren, jedoch auch in Dieselmotoren.

## Patentansprüche

1. Vorrichtung (110) zur Bestimmung einer Zusammensetzung eines Kraftstoffgemischs, insbesondere zur Bestimmung eines Ethanolanteils und/oder eines Wasseranteils in dem Kraftstoffgemisch, umfassend mindestens ein Gehäuse (116) mit mindestens einem elektrisch leitfähigen, von dem Kraftstoffgemisch durchströmbaren Gehäuseelement (128), wobei in das Gehäuseelement (128) mindestens ein Innenleiter (140) eingebracht ist, wobei der Innenleiter (140) von dem Gehäuseelement (128) zumindest teilweise umschlossen ist, wobei die Vorrichtung (110) weiterhin mindestens eine Verbindungsvorrichtung (146) zum Koppeln von Mikrowellensignalen aufweist, wobei das Gehäuseelement (128) mindestens einen den Innenleiter (140) zumindest teilweise umschließenden und elektrisch leitend mit dem Innenleiter (140) verbunden Becher (130) aufweist,
**dadurch gekennzeichnet, dass** die Vorrichtung (110) weiterhin mindestens einen von dem Kraftstoffgemisch in einer Strömungsrichtung (112) durchströmbaren Strömungsrohrabschnitt (118) umfasst, wobei das Gehäuse (116) den Strömungsrohrabschnitt umfasst, wobei der Innenleiter (140) und das Gehäuseelement (128) quer, vorzugsweise im Wesentlichen senkrecht, zu der Strömungsrichtung (112) in den Strömungsrohrabschnitt (118) eingebracht sind, und dass die Vorrichtung (110) weiterhin eine Einsteckkartusche (126) umfasst, wobei die Einsteckkartusche (126) den Innenleiter (140) und das Gehäuseelement (128) umfasst und in einem Messbereich (114) quer zur Strömungsrichtung (112) in eine Aufnahme (122) des Strömungsrohrabschnitts (118) eingesteckt ist, wobei das Gehäuseelement (128) und der Innenleiter (140) in einem Messbereich (114) aufgenommen sind, wobei ein Strömungsrohrquerschnitt in dem Messbereich (114) gegenüber einem Strömungsrohrquerschnitt außerhalb des Messbereichs (114) aufgeweitet ist und wobei die Aufweitung kontinuierlich durch Schalen (158) erfolgt, welche Bestandteil der Einsteckkartusche (126) sind.

2. Vorrichtung (110) nach dem vorhergehenden Anspruch, wobei das Gehäuseelement (128) den Innenleiter (140) koaxial umschließt.

3. Vorrichtung (110) nach Anspruch 1 oder 2, wobei der Becher (130) eine Mehrzahl von Bohrungen (138) aufweist, wobei die Bohrungen (138) einen Durchmesser aufweisen, welcher kleiner ist als die kleinste Wellenlänge der eingekoppelten Mikrowellen, insbesondere kleiner als 2 mm, vorzugsweise kleiner als 1 mm.

4. Vorrichtung (110) nach einem der vorhergehenden Ansprüche, wobei die Verbindungsvorrichtung (146) mindestens einen Koaxialstecker (148) aufweist, wobei der Koaxialstecker (148) mindestens einen Kontakt (150) zur Beaufschlagung des Innenleiters (140) mit Mikrowellensignalen umfasst.

5. Vorrichtung (110) nach Anspruch 1, wobei die Einsteckkartusche (126) mittels mindestens eines Dichtelements (154) gegenüber dem Strömungsrohrabschnitt (118) abgedichtet ist.

## Claims

1. Device (110) for determining a composition of a fuel mixture, in particular for determining an ethanol fraction and/or a water fraction in the fuel mixture, comprising at least one housing (116) having at least one electrically conductive housing element (128) through which the fuel mixture is able to flow, wherein at least one inner conductor (140) is inserted into the housing element (128), wherein the inner conductor (140) is at least partially surrounded by the housing element (128), wherein the device (110) furthermore has at least one connection device (146) for coupling microwave signals, wherein the housing element (128) has at least one cup (130) which at least partially surrounds the inner conductor (140) and which is connected in an electrically conductive manner to the inner conductor (140),
**characterized in that** the device (110) furthermore comprises at least one flow tube section (118) through which the fuel mixture is able to flow in a flow direction (112), wherein the housing (116) comprises the flow tube section, wherein the inner conductor (140) and the housing element (128) are inserted into the flow tube section (118) transverse, preferably substantially perpendicular, to the flow direction (112), and **in that** the device (110) furthermore comprises a plug-in cartridge (126), wherein the plug-in cartridge (126) comprises the inner conductor (140) and the housing element (128) and is plugged into a receiving part (122) of the flow tube section (118) in a measurement region (114) transverse to the flow direction (112), wherein the housing element (128) and the inner conductor (140) are received in a measurement region (114), wherein a flow tube cross section in the measurement region (114) is expanded in comparison with a flow tube cross section outside the measurement region (114), and wherein the expansion is realized continuously by shells (158) which are a constituent part of the plug-in cartridge (126).

2. Device (110) according to the preceding claim, wherein the housing element (128) surrounds the inner conductor (140) coaxially.

3. Device (110) according to Claim 1 or 2, wherein the cup (130) has a plurality of bores (138), wherein the bores (138) have a diameter which is smaller than the smallest wavelength of the microwaves coupled in, in particular smaller than 2 mm, preferably smaller than 1 mm.

4. Device (110) according to one of the preceding claims, wherein the connection device (146) has at least one coaxial plug (148), wherein the coaxial plug (148) comprises at least one contact (150) for applying microwave signals to the inner conductor (140).

5. Device (110) according to Claim 1, wherein the plug-in cartridge (126) is sealed off with respect to the flow tube section (118) by means of at least one sealing element (154).

## Revendications

1. Dispositif (110) pour la détermination d'une composition d'un mélange de carburant, en particulier pour la détermination d'une proportion d'éthanol et/ou d'une proportion d'eau dans le mélange de carburant, comprenant au moins un boîtier (116) avec au moins un élément de boîtier (128) électriquement conducteur pouvant être traversé par le mélange de carburant, dans lequel au moins un conducteur intérieur (140) est disposé dans l'élément de boîtier (128), dans lequel le conducteur intérieur (140) est entouré au moins partiellement par l'élément de boîtier (128), dans lequel le dispositif (110) présente en outre au moins un dispositif de raccordement (146) pour le couplage de signaux à microondes, dans lequel l'élément de boîtier (128) présente au moins un godet (130) entourant au moins en partie le conducteur intérieur (140) et en liaison électriquement conductrice avec le conducteur intérieur (140),
**caractérisé en ce que** le dispositif (110) comprend en outre au moins une partie de tube d'écoulement (118) pouvant être parcourue par le mélange de carburant dans une direction d'écoulement (112), dans lequel le boîtier (116) comprend la partie de tube d'écoulement, dans lequel le conducteur intérieur (140) et l'élément de boîtier (128) sont placés transversalement, de préférence essentiellement perpendiculairement à la direction d'écoulement (112) dans la partie de tube d'écoulement (118), et **en ce que** le dispositif (110) comprend en outre une cartouche à insérer (126), dans lequel la cartouche à insérer (126) comprend le conducteur intérieur (140) et l'élément de boîtier (128) et est insérée dans une région de mesure (114) transversalement à la direction d'écoulement (112) dans un logement (122) de la partie de tube d'écoulement (118), dans lequel l'élément de boîtier (128) et le conducteur intérieur (140) sont logés dans une région de mesure (114), dans lequel une section transversale du tube d'écoulement est élargie dans la région de mesure (114) par rapport à une section transversale du tube d'écoulement à l'extérieur de la région de mesure (114) et dans lequel l'élargissement est réalisé par des coquilles (158), qui font partie de la cartouche à insérer (126).

2. Dispositif (110) selon la revendication précédente, dans lequel l'élément de boîtier (128) entoure coaxialement le conducteur intérieur (140).

3. Dispositif (110) selon la revendication 1 ou 2, dans lequel le godet (130) présente une multiplicité de trous (138), dans lequel les trous (138) présentent un diamètre, qui est plus petit que la plus petite longueur d'onde des microondes couplées, en particulier plus petit que 2 mm, de préférence plus petit que 1 mm.

4. Dispositif (110) selon l'une quelconque des revendications précédentes, dans lequel le dispositif de raccordement (146) présente au moins un connecteur coaxial (148), dans lequel le connecteur coaxial (148) comprend au moins un contact (150) pour alimenter le conducteur intérieur (140) avec des signaux à microondes.

5. Dispositif (110) selon la revendication 1, dans lequel la cartouche à insérer (126) est rendue étanche par rapport à la partie de tube d'écoulement (118) au moyen d'au moins un élément d'étanchéité (154).
